# EUROPÄISCHE PATENTSCHRIFT

(11) **EP 3 663 284 B1**
(45) Veröffentlichungstag und Bekanntmachung des Hinweises auf die Patenterteilung: **03.02.2021**
(21) Anmeldenummer: 18210919.9
(22) Anmeldetag: 07.12.2018
(51) Int. Cl.: C07D 211/02, C07D 211/74

(54) **VERBESSERTES VERFAHREN ZUR HERSTELLUNG VON TRIACETONAMIN**
IMPROVED METHOD FOR THE PREPARATION OF TRIACETONAMINE
PROCÉDÉ AMÉLIORÉ DE PRODUCTION DE LA TRIACÉTONAMINE

(43) Veröffentlichungstag der Anmeldung: 10.06.2020
(73) Patentinhaber: Evonik Operations GmbH, 45128 Essen (DE)
(72) Erfinder: MINKE, Katharina, 45130 Essen (DE); RIEB, Julia, 45772 Marl (DE); NEUMANN, Manfred, 45770 Marl (DE)
(74) Vertreter: Evonik Patent Association

(56) Entgegenhaltungen:
- CN-A- 108 383 704

## Beschreibung

Die vorliegende Erfindung betrifft ein verbessertes Verfahren zur Herstellung von Triacetonamin. Dabei erfolgt eine Behandlung des Rohproduktes aus der Triacetonaminherstellung, welche zu einem Anstieg des Gehalts an mit Ammoniak leicht reagierbaren Verbindungen führt. Dieses Verfahren erlaubt effizientes Recycling der bei der Synthese von Triacetonamin entstehenden Nebenprodukte.

### Hintergrund der Erfindung

Triacetonamin (2,2,6,6-Tetramethyl-4-piperidinon; CAS-Nummer: 826-36-8; im Folgenden "TAA") ist ein wichtiges chemisches Intermediat, welches zur Synthese zahlreicher Folgeprodukte wie beispielsweise Lichtstabilisatoren (*hindered amine light stabilizers*; [HALS]), Oxidationsmitteln und Polymerisationsmoderatoren (z. B. Nitroxyl-Radikale) eingesetzt wird.

Die Herstellung von Triacetonamin aus Aceton und Ammoniak ist in Form verschiedener Verfahren dokumentiert. Dabei gliedern sich die Herstellungsverfahren in die direkte (einschrittige) Synthese von TAA aus den Edukten, beispielsweise beschrieben in DE 24 29 937 A1, US 4,536,581 A, JPS54-88275 A oder in Zeitschrift für Naturforschung 1976, 328 - 337 und 338 - 345, sowie die indirekte (zweistufige) Synthese über Acetonin (2,2,4,4,6-Pentamethyl-1,2,5,6-tetrahydro-pyrimidin), beispielsweise beschrieben in DE 24 29 935 A1 oder DE 24 29 936 A1, oder über Phoron (2,6-Dimethyl-2,5-heptadien-4-on), z. B. beschrieben in DE 2 352 127 A1. Bei der zweistufigen TAA-Synthese über Acetonin wird dies zunächst ausgehend von Aceton und Ammoniak gebildet, und reagiert dann in einem anschließenden Schritt unter Abspaltung von einem Äquivalent Ammoniak weiter zu TAA. Im Falle des Syntheseverfahrens über Acetonin werden jedoch immer beide Spezies (TAA und Acetonin) gleichzeitig gebildet, die Acetoninbildung ist allerdings kinetisch gegenüber der TAA-Bildung stark bevorzugt. In der "einstufigen" TAA-Synthese wird Acetonin lediglich nicht isoliert.

Die Herstellung von TAA ist grundsätzlich sowohl homogen katalysiert (meist durch Ammoniumsalze) als auch heterogen katalysiert (z. B. an sauren Ionentauschern) möglich.

Die meisten Schriften aus dem Stand der Technik beziehen sich auf homogen katalysierte Reaktionen. Am häufigsten werden dabei Calciumchlorid (z.B. in Chemical Industries 2003, 89, 559-564; Zeitschrift für Naturforschung 1976, 328 - 337 und 338 - 345), Ammoniumchlorid (z. B. in JP 2003-206277 A; JP 2001-31651 A; JPH4-154762 A) und Hydrazinderivate (z. B. in JPS54-88275 A, JPS54-112873 A) genannt. Bei Einsatz dieser Katalysatoren treten allerdings Probleme auf. So hat etwa der Einsatz von Calciumchlorid den Nachteil, dass eine sehr langsame Reaktion erfolgt. Im Falle des Ammoniumchlorids ist die Reaktionsgeschwindigkeit höher, das eingesetzte Chlorid zeigt allerdings eine hohe Korrosivität gegenüber Stahl. Hydrazinderivate zeigen demgegenüber eine sehr hohe Toxizität.

Demgegenüber wurden auch Reaktionen an heterogenen Katalysatoren beschrieben, wie zum Beispiel in der DE 28 07 172 A1 und der CN 103224465 A.

Im Allgemeinen wird TAA in einer Matrix hergestellt, bei der das Aceton in großem Überschuss vorliegt und sowohl als Reaktionspartner als auch als Lösungsmittel dient. Daher ergibt sich am Ende der Reaktion ein Rohprodukt, welches neben TAA einen großen Anteil an Aceton, nicht umgesetzten Ammoniak, durch die Kondensation gebildetes Wasser und bei homogenkatalytischen Verfahren den Katalysator enthält. Darüber hinaus sind weitere Nebenkomponenten enthalten, wie z. B. acyclische Kondensationsprodukte (z. B. Diacetonalkohol, Diacetonamin, Mesityloxid, Phoron etc.), cyclische Kondensationsprodukte [z. B. Acetonin, 2,2,4,6-Tetramethyl-2,3-dihydropyridin (im Folgenden "TMDH-Pyridin")] oder höhermolekulare Kondensationsprodukte ("Hochsieder").

Einige acyclische Additions- und Kondensationsprodukte (z. B. Diacetonalkohol [4-Hydroxy-4-methylpentan-2-on], Diacetonamin [4-Amino-4-methylpentan-2-on], Mesityloxid [4-Methylpent-3-en-2-on], Phoron etc.) können ihrerseits anstatt Aceton als Edukte mit Ammoniak zu TAA umgesetzt werden, was man sich beispielsweise in Verfahren mit einem prozessinternen Recyclingstrom zunutze macht (DE 28 07 172 A1).

Im Stand der Technik sind daneben TAA-Synthesen ausgehend von Aceton oder auch diesen Aceton-Kondensationsprodukten beschrieben.

CN 108383704 A beschreibt die batchweise Verwendung der bei der Synthese des TAA entstehenden Nebenprodukte zur Herstellung von Aceton, welches dann wiederum als Startmaterial eingesetzt werden kann. Dabei wird in Anwesenheit eines Katalysators Wasser zum Nebenproduktegemisch zugegeben und die Nebenprodukte gespalten.

CN 108484483 A beschreibt ein Verfahren zur Reinigung von TAA, welches Destillation und Kristallisation kombiniert.

CN 108383776 A beschreibt die Abtrennung und Wiederverwendung der bei der Synthese von TAA entstehenden Nebenprodukte in folgenden TAA-Syntheseschritten.

Die Verwendung dieser Nebenprodukte als Ausgangsstoffe bringt jedoch einige Probleme mit sich. Einerseits verfügen diese über eine geringere Reaktivität und reagieren zum Teil sehr viel langsamer als Aceton. Allein Mesityloxid zeigt eine mit Aceton vergleichbare Reaktivität. Die Verwendung dieser Verbindungen als Ausgangsstoffe für die Synthese weiteren TAAs ist deshalb gerade in großtechnischen Verfahren nachteilig.

Andererseits besteht ein weiterer Nachteil der herkömmlichen Verfahren zur TAA-Synthese darin, dass die beschriebenen Nebenprodukte aufwendig von überschüssigem Aceton und dem gewünschten Produkt TAA abzutrennen sind. Die Siedepunkte der typischen Nebenprodukte Diacetonalkohol (CAS-Nummer 123-42-2; Siedepunkt bei Normaldruck: 166 °C), Acetonin (CAS-Nummer 556-72-9; Siedepunkt ∼ 170 °C), Diacetonamin (CAS-Nummer 625-04-7, Siedepunkt ∼ 180 °C), Phoron (CAS-Nummer 504-20-1; Siedepunkt bei Normaldruck: 197 °C) liegen zwischen den Siedepunkten des Acetons (CAS-Nummer 67-64-1; Siedepunkt bei Normaldruck: 56 °C) bzw. Mesityloxids (CAS-Nummer 141-79-7; Siedepunkt bei Normaldruck: 130 °C) und des TAAs (Siedepunkt bei Normaldruck: 205 °C), und im Falle des Isophorons (CAS-Nummer 78-59-1; Siedepunkt bei Normaldruck: 215 °C) auch darüber.

Dadurch ist die Reinigung des TAAs aus dem erhaltenen Reaktionsgemisch kompliziert. Bei der destillativen Reinigung sind viele Destillationsstufen nötig sind, um das gewünschte Produkt TAA möglichst rein und vollständig von den Nebenprodukten abzutrennen. Dies wiederum erfordert aufwendige Destillationsapparaturen, wie zum Beispiel eine Kolonne mit einer hohen Anzahl theoretischer Böden.

Es bestand deshalb der Bedarf nach einem effizienten Verfahren zur Synthese von TAA, welches die vorgenannten Probleme nicht aufweist und insbesondere ein möglichst reines Produkt TAA liefert, wobei gleichzeitig die effiziente Wiederverwertung der in der TAA-Synthese erhaltenen Nebenprodukte ermöglicht werden soll.

### Detaillierte Beschreibung der Erfindung

Die vorgenannte Aufgabe wird mittels der vorliegenden Erfindung gelöst. Es wurde nämlich überraschend festgestellt, dass ein besonders effizientes Verfahren zur Herstellung von TAA dadurch ermöglicht wird, dass die bei der TAA-Herstellung aus Aceton und Ammoniak erhaltenen Nebenprodukte mit Wasser umgesetzt und das Rohprodukt so an Spezies, die in der Reaktion mit Ammoniak zu weiterem TAA besonders reaktiv sind, angereichert wird. Es hat sich außerdem überraschenderweise gezeigt, dass die destillative Abtrennung des Acetons und anderer angereicherter Spezies wie Mesityloxid aus dem so behandelten Rohprodukt viel effizienter durchgeführt werden kann und diese so besser recycelt werden können.

Dies war umso überraschender, als das Rohprodukt aus der Umsetzung von Ammoniak mit Aceton zu TAA Wasser umfasst. Um die vorteilhaften Effekt der Erfindung zu verwirklichen, ist jedoch die Zugabe zusätzlichen Wassers nötig.

Das erfindungsgemäße Verfahren ist demnach eines zur Herstellung von Triacetonamin umfassend die folgenden Schritte:
(a) Umsetzung von Aceton und Ammoniak in Gegenwart eines Katalysators **K1** zu einem Rohprodukt **RP1** umfassend Triacetonamin, Aceton und Mesityloxid, und mindestens ein Nebenprodukt ausgewählt aus der Gruppe bestehend aus Diacetonalkohol, Diacetonamin, Acetonin, Phoron, Isophoron,
(b) mindestens teilweise destillative Abtrennung von Aceton und/oder Mesityloxid aus **RP1**,
(c) Zugabe von Ammoniak und gegebenenfalls weiteren Acetons zu dem in Schritt (b) aus **RP1** abgetrennten Aceton und/oder Mesityloxid und mindestens teilweise Umsetzung des zugegebenen Ammoniaks mit Aceton und/oder Mesityloxid zu TAA in Gegenwart eines Katalysators **K2**, wodurch ein Rohprodukt **RP2** umfassend Triacetonamin erhalten wird,
   dadurch gekennzeichnet, dass
(d) vor und/oder während der in Schritt (b) stattfindenden destillativen Abtrennung von Aceton und/oder Mesityloxid aus **RP1** in Schritt (b) Wasser zu **RP1** zugegeben wird und mindestens eines der Nebenprodukte in **RP1** ausgewählt aus der Gruppe bestehend aus Diacetonalkohol, Diacetonamin, Acetonin, Phoron, Isophoron mindestens teilweise mit Wasser umgesetzt wird, so dass mindestens eines der Nebenprodukte in **RP1** mindestens teilweise in Mesityloxid und/oder Aceton gespalten wird.

### 1. Schritt (a)

Im Schritt (a) des erfindungsgemäßen Verfahrens werden Aceton und Ammoniak in Gegenwart eines Katalysators **K1** umgesetzt.

Dabei wird ein Rohprodukt **RP1** umfassend Triacetonamin erhalten. Dieses umfasst neben dem gewünschten Produkt TAA auch nicht reagiertes Aceton sowie als Nebenprodukt Mesityloxid. Mesityloxid ist das einfachste Kondensationsprodukt, das stets zumindest in geringer Menge bei der Umsetzung von Ammoniak und Aceton zu TAA durch Aldolkondensation zweier Moleküle Aceton gebildet wird. Auch höhermolekulare Kondensationsprodukte des Acetons mit sich oder Ammoniak entstehen bei der Umsetzung im Schritt (a) und sind deshalb vom Rohprodukt **RP1** umfasst. Dies sind vor allem Nebenprodukte ausgewählt aus der Gruppe bestehend aus Diacetonalkohol, Diacetonamin, Acetonin, Phoron, Isophoron, bevorzugt ausgewählt aus der Gruppe bestehend aus Diacetonalkohol, Diacetonamin, Acetonin, Isophoron.

Die Umsetzung in Schritt (a) wie auch das gesamte erfinderische Verfahren können kontinuierlich oder im Batch-Betrieb durchgeführt werden.

Im Falle des Batch-Betriebs werden bevorzugt alle Edukte zusammengegeben, dann wird die Reaktionsmischung erwärmt. Als Reaktor kommen dabei alle üblichen Reaktortypen in Frage, z.B. Rührreaktoren, Schlaufenreaktoren oder Reaktoren mit innenliegenden Wärmetauschern.

Im kontinuierlichen Betrieb werden bevorzugt alle Chemikalien gleichzeitig bei der Reaktionstemperatur zudosiert. Bei der kontinuierlichen Reaktion kann jeder dem Fachmann bekannte Reaktor eingesetzt werden, z.B. ein kontinuierliches Strömungsrohr, ein kontinuierlicher Rührkessel, eine Rührkesselkaskade, sowie mögliche Kombinationen aus diesen einzelnen Elementen. Dabei wird bevorzugt eine Kombination von einem oder mehreren Reaktoren mit internem oder externem Kreislauf, gefolgt von einem Nachreaktor mit Strömungsrohrcharakteristik, eingesetzt.

Die Reaktionszeit in Schritt (a) im Batch-Betrieb liegt im Bereich von 1 bis 15 Stunden, bevorzugt im Bereich von 3 bis 9 Stunden, besonders bevorzugt im Bereich von 5 bis 7 Stunden. Die Reaktionszeiten für die kontinuierliche Verfahrensführung ergeben sich durch die Gesamt-Verweilzeit der Reaktanden im Reaktor und liegen in dem für den Batch-Betrieb genannten Bereich.

Die *"volume space velocity"* für Ammoniak im kontinuierlichen Betrieb liegt in Schritt (a) insbesondere bei 0.01 bis 124.20 h⁻¹, bevorzugt 0.03 bis 5.25 h⁻¹, am bevorzugtesten bei 0.06 h⁻¹ (dies entspricht dem Volumen an Ammoniak, was pro Stunde und pro Volumen des Reaktors durch diesen Reaktor fließt, abgekürzt als "LHSV").

Die *"volume space velocity"* für Aceton im kontinuierlichen Betrieb liegt in Schritt (a) insbesondere bei 0.15 bis 1.33 h⁻¹, bevorzugt 0.66 bis 1.17 h⁻¹ (dies entspricht dem Volumen an Aceton, was pro Stunde und pro Volumen des Reaktors durch diesen Reaktor fließt).

Die Umsetzung in Schritt (a) kann in Gegenwart weiterer Lösungsmittel oder nur in Aceton, also ohne Zugabe weiterer Lösungsmittel, stattfinden. In den Fällen, in denen ein Lösungsmittel in Schritt (a) eingesetzt wird, können sämtliche Lösungsmittel eingesetzt werden, die die Reaktion nicht verhindern. Insbesondere sind die möglichen Lösungsmittel aliphatische Lösungsmittel, bevorzugt Pentan, Hexan, Heptan, Octan, Decan, Cyclohexan, Tetramethylsilan; aromatische Lösungsmittel, bevorzugt Benzol, Toluol, Xylol; Ether-Verbindungen, bevorzugt Diethylether, Dipropylether, Dibutylether, Methyl-*tert*-Butylether; halogenierte Lösungsmittel, bevorzugt Dichlormethan, Chloroform, Tetrachlormethan; Alkohole, bevorzugt Methanol, Ethanol, Propanol, Isopropanol, Butanol, *tert*-Butanol; Ester, bevorzugt Methylacetat, Ethylacetat, Propylacetat, Butylacetat. Besonders bevorzugt findet die Umsetzung in Schritt (a) in Aceton, ohne Zugabe weiterer Lösungsmittel, statt.

Die Umsetzung in Schritt (a) wird vorzugsweise bei erhöhter Temperatur durchgeführt, insbesondere bei Temperaturen im Bereich von 20 °C bis 180°C, bevorzugt im Bereich von 40 °C bis 100 °C, bevorzugter im Bereich von 55 °C bis 90°C, noch bevorzugter im Bereich von 60 °C bis 90 °C, am bevorzugtesten bei 75 °C.

Die Umsetzung in Schritt (a) wird insbesondere entweder bei Eigendruck der Komponenten oder bei erhöhtem Druck durchgeführt. So wird die Umsetzung in Schritt (a) bevorzugt bei einem Druck im Bereich von 1 bis 16 bar, bevorzugter bei einem Druck im Bereich von 1 bis 15 bar, noch bevorzugter bei einem Druck im Bereich von 7 bis 14 bar, noch viel mehr bevorzugter bei 12 bar durchgeführt werden.

Ammoniak wird im Schritt (a) des erfindungsgemäßen Verfahren bevorzugt als Reinstoff, d. h. als Gas zudosiert und liegt insbesondere während der Reaktion gelöst in Aceton bzw. gelöst im Reaktionsgemisch vor.

Aceton wird im Schritt (a) bevorzugt als Reinstoff zudosiert. Alternativ kann Aceton verwendet werden, was in Schritt (b) einer vorher durchlaufenen Runde des erfindungsgemäßen Verfahrens abgetrennt wurde und auf diese Weise rezykliert wird. Es versteht sich von selbst, dass dann in der Reaktionslösung im Schritt (a) neben Aceton und Ammoniak Kondensationsprodukte des Acetons mit sich selbst oder Ammoniak vorliegen können. Diese können aus vorhergehenden Umsetzungsschritten von Ammoniak und Aceton zu TAA stammen.

Der Schritt (a) des erfindungsgemäßen Verfahrens wird in Gegenwart eines Katalysators **K1** durchgeführt. Als Katalysator **K1** sind alle im Stand der Technik für diesen Reaktionstyp genannten Katalysatoren einsetzbar. Der Katalysator **K1** kann dabei homogen oder heterogen sein, ist bevorzugt jedoch heterogen.

Als homogener Katalysator **K1** sind alle im Stand der Technik für diesen Reaktionstyp beschriebenen homogenen Katalysatoren geeignet, z. B. Brönstedt-Säuren, Salze dieser Säuren oder Lewissäuren, wie sie in der EP 2 706 056 A1 beschrieben sind.

Der Begriff "Brönsted-Säuren" im Sinne der Erfindung umfasst insbesondere Salzsäure, Schwefelsäure, Salpetersäure, organische Säuren (RCOOH) oder Sulfonsäuren (RSO₃H), wobei R ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten. Substituierte Kohlenwasserstoffreste im Sinne der Erfindung sind Kohlenwasserstoffreste, die mit Heteroatomen substituiert sind, insbesondere Kohlenwasserstoffreste, welche mit einem oder mehreren -OH, -NH, -CN, Alkoxy- und/oder Halogenresten substituiert sind, bevorzugt mit einem oder mehreren Halogenresten substituiert sind, besonders bevorzugt mit einem oder mehreren Resten ausgewählt aus F, Cl, Br und I substituiert sind, ganz besonders bevorzugt mit einem oder mehreren Resten ausgewählt aus F und Cl substituiert sind.

"Salze einer Brönstedtsäure" im Sinne der Erfindung sind insbesondere Ammoniumsalze (d. h. Salze mit Ammoniak, Aminen, Hydrazinen, Hydroxylaminen) oder Phosphoniumsalze (d. h. Salze mit Phosphanen). Lewis-Säuren im Sinne der Erfindung sind insbesondere Verbindungen der 4. bzw. der 13. Gruppe des Periodensystems, bevorzugt Halogenide (AlCl₃, BF₃, TiCl₄), Alkoxide [Al(OR*)₃, B(OR*)₃, Ti(OR*)₄] oder Alkylverbindungen (z.B. AlR*₃), wobei R* ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten.

Lewis-Säuren im Sinne der Erfindung sind auch Salze Lewis-saurer Alkali- bzw. Erdalkalimetalle (z.B. CaCl₂, MgCl₂, LiCI).

Vorzugsweise ist in den Fällen, in denen **K1** ein homogene Katalysator ist, dieser ausgewählt aus der Gruppe der Ammoniumsalze, insbesondere aus der Gruppe umfassend Salze aus Ammoniak und starken Brönsted-Säuren [z. B. Salzsäure, Schwefelsäure, Salpetersäure, organische Säuren (R**COOH) oder Sulfonsäuren (R**SO₃H), wobei R** ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten].

Bevorzugt ist in den Fällen, in denen **K1** ein homogener Katalysator ist, dieser dann Ammoniumnitrat. Ammoniumnitrat hat den Vorteil, dass es günstig, nicht toxisch, halogenidfrei und damit weniger korrosiv ist.

Bevorzugt wird als Katalysator **K1** jedoch ein heterogener Katalysator, insbesondere ein fester, saurer Katalysator eingesetzt, wie er beispielsweise in der DE 28 07 172 A1, der CN 103224465 A oder der DE 10 2010 062 804 A1 beschrieben ist. Dies sind solche Katalysatoren, die in dem Reaktionsmedium praktisch unlöslich sind. Bevorzugt wird dazu ein Katalysator genutzt, der anorganisch oder organisch ist und aktive saure Gruppen, bevorzugt Sulfonsäureestergruppen oder Phosphorsäureestergruppen, aufweist.

**K1** ist demnach insbesondere ausgewählt aus der Gruppe bestehend aus Aluminiumhydrosilikate vom Betonit- und/oder Montmorillonit-Typ, anorganische Ionenaustauscher auf Aluminiumsilikat-Basis vom Zeolithtyp, Mordenittyp, Erionittyp oder auch mit Phosphorsäure bei 700 bis 1100 °C, wie in CA 772 201 beschrieben, behandelte Diatomerde.

Besonders für **K1** bevorzugte heterogene Katalysatoren sind lonenaustauscherharze, insbesondere Kationenaustauscherharze. Diese sind bevorzugt sauer.

Als lonenaustauscherharze für **K1** kommen insbesondere solche auf anorganischer (beispielsweise Siliciumdioxid) oder organischer (beispielsweise Polystyrol oder Polystyrolcopolymere, wie Styrol-Divinylbenzol-Copolymere), bevorzugt organischer, Basis, die protische Säuregruppen, insbesondere Alkylsulfonsäureestergruppen, Sulfonsäureestergruppen (-SO₃⁻), Phosphorsäureestergruppen, insbesondere Sulfonsäureestergruppen aufweisen, in Frage.

Ein besonders bevorzugter heterogener Katalysator für **K1** ist ausgewählt aus der folgenden Gruppe:
- Ein Katalysator, der ein Polymerharz, insbesondere Polystyrol oder Styrol-Divinylbenzol-Copolymer, bevorzugt Polystyrol, und protische Säuren, insbesondere -SO₃⁻-Gruppen, als funktionelle Gruppen aufweist (käuflich erwerblich als "Amberlyst® 15", "Amberlite® 200", "Lewatit® SP 120" bzw. "Lewatit® K2621"); daneben ist auch Polystyrolsulfonat mit der CAS-Nummer: 28210-41-5 einsetzbar;
- Ein Katalysator, der protische Säuren, insbesondere Sulfonsäure in polymerer Form aufweist und perfluoriert sein kann (beschrieben in DE 10 2010 062 804 A1, US 4,831,146). Dieser kann zum Beispiel ein sulfoniertes Tetrafluorethylen (CAS-Nummer: 31175-20-9) oder eine feste geträgerte perfluorierte Sulfonsäure in polymerer Form mit Siliciumdioxid als Trägermaterial sein. Derartige Katalysatoren sind unter anderem unter den Handelsnamen Nafion®, Aciplex® F, Femion®, Neosepta®, Fumion® F erhältlich. Ein bevorzugter Katalysator ist Nafion® SAC-13. Bei Nafion® SAC-13 handelt es sich um poröse Siliciumdioxidpartikel, auf dem Nafion® in einer Beladung von etwa 13 Gew.-% adsorbiert worden ist;
- Poly(2-acrylamido-2-methyl-1-propanesulfonsäure), vertrieben als PolyAMPS® von Lubrizol.

Am bevorzugtesten wird als **K1** ein heterogener Katalysator eingesetzt, der ein Polymerharz, insbesondere Polystyrol oder Styrol-Divinylbenzol-Copolymer, bevorzugt Polystyrol, und protische Säuren, insbesondere -SO₃⁻-Gruppen, als funktionelle Gruppen aufweist (käuflich erwerblich als "Amberlyst® 15", "Amberlite® 200", "Lewatit® SP 120" bzw. "Lewatit® K2621").

Die Einsatzverhältnisse der Edukte im Schritt (a) des erfindungsgemäßen Verfahrens können in weiten Bereichen gewählt werden, insbesondere wird Aceton im Überschuss zu Ammoniak eingesetzt. Bevorzugt beträgt das Molverhältnis von in Schritt (a) eingesetztem Aceton zu in Schritt (a) eingesetztem Ammoniak 3 : 1 bis 20 : 1, wobei ein Verhältnis von 6 : 1 bis 10 : 1 bevorzugt ist und ein Verhältnis von 7 : 1 besonders bevorzugt ist.

Die eingesetzte Menge an Katalysator **K1** ist nicht besonders beschränkt und kann vom Fachmann bestimmt werden. Typischerweise kann, wenn der Katalysator ein homogener aus der Gruppe der Brönstedt-Säuren, Salze dieser Säuren oder Lewissäuren, bevorzugt ein Ammoniumsalz, noch bevorzugter Ammoniumnitrat ist, dieser im Molverhältnis von Ammoniak zu Katalysator, vorzugsweise Ammoniumnitrat, im Bereich von 1 : 0.8 bis 1 : 0.02 eingesetzt werden. Ganz besonders bevorzugt liegt das Molverhältnis von Aceton : Ammoniak : Ammoniumnitrat im Bereich von 7 bis 8 : 0.9 bis 1.1 : 0.085 bis 0.098.

In der bevorzugten Ausführungsform, in welcher ein fester, saurer Ionenaustauscher für **K1** eingesetzt wird, kann dieser als Festbett eingesetzt werden, beispielsweise in einer Menge von 10 bis 20 Vol.-% bezogen auf die Gesamtmenge des in Schritt (a) eingesetzten Acetons und - falls ein solches eingesetzt wurde - des Mesityloxids.

Im Anschluss an Schritt (a) des erfindungsgemäßen Verfahrens wird dann ein Rohprodukt **RP1** erhalten, welches neben dem gewünschten Produkt Triacetonamin auch das ursprünglich eingesetzte Edukt Aceton, sowie als Nebenprodukt Mesityloxid und mindestens ein Nebenprodukt ausgewählt aus der Gruppe bestehend aus Diacetonalkohol, Diacetonamin, Acetonin, Phoron, Isophoron, bevorzugt ausgewählt aus der Gruppe bestehend aus Diacetonalkohol, Diacetonamin, Acetonin, Phoron, Isophoron, umfasst.

Der Anteil des TAA, Aceton, Mesityloxid, Wasser und der genannten Nebenprodukte in **RP1** ist nicht weiter beschränkt und ergibt sich aus der Stöchiometrie und den speziellen Reaktionsbedingungen. Der Anteil der jeweiligen Verbindung kann per GC bestimmt werden. Beispielsweise liegt nach der Reaktion ein Gemisch vor, in dem der Gehalt an Aceton bei 55 bis 60 Gew.-%, an Mesityloxid bei 10 Gew.-%, an TMDH-Pyridin bei 5 bis 6 Gew.-%, an der Summe aus Diacetonamin, Aicetonalkohol und Phoron bei 4 bis 6 Gew.-%, an TAA bei 14 bis 16 Gew.-%, und an höher als TAA siedenden Komponenten (zum Beispiel Iso-Phoron) bei 3 bis 4 Gew.-% liegt, und der Anteil von Wasser 7 Gew.-% ist.

### 2. Schritt (b)

Im Schritt (b) des erfindungsgemäßen Verfahrens werden mindestens teilweise Aceton und/oder Mesityloxid aus **RP1** abgetrennt. Dies erfolgt destillativ, bevorzugt in einer Destillationskolonne.

Gegebenenfalls findet während oder vor, bevorzugt vor, dem Schritt (b) auch eine Abtrennung des Katalysators **K1** statt. Dies kann durch Zugabe einer Base geschehen. Zum Beispiel wird NaOH zugegeben, wenn **K1** ein Ammoniumsalz ist, und das dann ausgefallene Natriumnitrat wird anschließend abgetrennt.

Bei Verwendung eines heterogenen Katalysators erübrigt sich ein separater Reinigungsschritt oder ist zumindest deutlich einfacher, da beispielsweise bei Verwendung eines Festbettkatalysators dieser Katalysator im Reaktor verbleibt oder in anderen Fällen im Reaktionskessel verbleiben kann und/ oder durch Filtration abgetrennt werden kann. Auch deshalb ist die Verwendung eines heterogenen Katalysators für **K1** vorzuziehen.

### 3. Schritt (d)

Die Erfindung beruht nun drauf, dass vor und/oder während des Schrittes (b), also der destillativen Abtrennung von Aceton und/oder Mesityloxid aus **RP1**, Wasser zu **RP1** zugegeben wird und mindestens eines der Nebenprodukte in **RP1** ausgewählt aus der Gruppe bestehend aus Phoron, Diacetonalkohol, Diacetonamin, Acetonin, Isophoron, bevorzugt ausgewählt aus der Gruppe bestehend aus Diacetonalkohol, Diacetonamin, Acetonin, Isophoron, mindestens teilweise mit Wasser umgesetzt werden, so dass mindestens eines der Nebenprodukte in **RP1** mindestens teilweise in Mesityloxid und/oder Aceton gespalten wird. Im Folgenden wird dieser Schritt als "Schritt (d)" bezeichnet.

Der Schritt (d) des erfindungsgemäßen Verfahrens gewährleistet, dass mindestens eines der reaktionsträgeren Nebenprodukte im **RP1**, also mindestens eines ausgewählt aus der Gruppe bestehend aus Diacetonalkohol, Diacetonamin, Acetonin, Phoron, Isophoron, bevorzugt mindestens einer ausgewählt aus der Gruppe bestehend aus Diacetonalkohol, Diacetonamin, Acetonin, Isophoron, zu Aceton und/oder Mesityloxid umgesetzt wird. Dies führt dazu, dass diese mittelsiedenden (im Sinne von "zwischen Mesityloxid und TAA siedend") bzw. höhersiedenden (im Sinne von "höher als TAA siedend") Nebenprodukte, wie zum Beispiel Isophoron, zu niedrigsiedendem Mesityloxid und Aceton zersetzt werden, was insbesondere die destillative Reinigung des TAA aus dem Rohprodukt **RP1** beträchtlich vereinfacht.
Zusätzlich dazu können die reaktivere Spezies Aceton und dessen reaktivstes Äquivalent Mesityloxid vorteilhafter wieder in den nachfolgenden Schritten mit Ammoniak zu weiterem TAA umgesetzt werden, und zwar deutlich besser als wenn stattdessen die mittelsiedenden und höhersiedenden Nebenprodukte mit Ammoniak in einem anschließenden Schritt zu weiterem TAA umgesetzt werden müssten.

Die Zugabe von Wasser zu **RP1** erfolgt gemäß Schritt (d) vor und/oder während der in Schritt (b) stattfindenden destillativen Abtrennung von Aceton und/oder Mesityloxid aus **RP1**. Es versteht sich von selbst, dass die Zugabe von Wasser "vor der Abtrennung von Aceton und/oder Mesityloxid aus **RP1**" bedeutet, dass dieser Zeitpunkt zwischen den Schritten (a) und (b) liegt, da erst nach Schritt (a) das Rohprodukt **RP1** erhalten wird.

Die Zugabe von Wasser in Schritt (d) ist dabei wesentlich für die Durchführung der Erfindung, da dadurch das Gleichgewicht von der Seite der Nebenprodukte auf die der Spaltprodukte derselben, also neben Aceton vor allem Mesityloxid, verschoben wird. Um diese Verschiebung des Gleichgewichts ausreichend zu gewährleisten, genügt nicht das Wasser, welches aus der Umsetzung in Schritt (a) resultiert und noch mindestens teilweise in **RP1** vorliegen kann. Es ist vielmehr nötig, im Schritt (d) zumindest eine bestimmte Menge zusätzlichen Wassers zu **RP1** zu geben, um das Gleichgewicht in der folgenden Umsetzung auf die Seite des gewünschten Produkts Aceton und/oder Mesityloxid zu schieben.

Insbesondere wird im Schritt (d) des erfindungsgemäßen Verfahrens Wasser in einer Menge von ≥ 0.1 Gewichts.-%, bevorzugt ≥ 0.5 Gew.-%, bevorzugter ≥ 1 Gew.-%, noch bevorzugter ≥ 5 Gew.-%, noch mehr bevorzugter im Bereich von 5 bis 40 Gew.-%, noch viel mehr bevorzugter im Bereich von 10 bis 20 Gew.-% zugegeben, jeweils bezogen auf die Summe der Gewichte des von **RP1** umfassten Phorons, Diacetonalkohols, Diacetonamins, Acetonins und Isophorons. Dieser Anteil kann vom Fachmann mit Hilfe von Gaschromatographie ermittelt werden. Das Gesamtgewicht von **RP1** kann bei kontinuierlichen Verfahren durch Ermittlung des Durchflusses (Massedurchflußmesser), bei Batchverfahren durch Auswiegen ermittelt werden.

Alternativ kann im Schritt (d) des erfindungsgemäßen Verfahrens Wasser in einer Menge von ≥ 1 Gewichts.-%, bevorzugt ≥ 3 Gew.-%, bevorzugter ≥ 4 Gew.-%, noch bevorzugter ≥ 5 Gew.-%, noch mehr bevorzugter im Bereich von 5 bis 40 Gew.-%, noch viel mehr bevorzugter im Bereich von 10 bis 20 Gew.-%, jeweils bezogen auf das Gesamtgewicht der in Schritt (a) des erfindungsgemäßen Verfahrens eingesetzten Menge an Aceton, zugegeben werden.

Die Umsetzung des zugegebenen Wassers in Schritt (d) mit mindestens einem der Nebenprodukte in **RP1** ausgewählt aus der Gruppe bestehend aus Phoron, Diacetonalkohol, Diacetonamin, Acetonin, Isophoron, bevorzugt ausgewählt aus der Gruppe bestehend aus Diacetonalkohol, Diacetonamin, Acetonin, Isophoron, zu Aceton und/oder Mesityloxid kann dann unter den dem Fachmann geläufigen Bedingungen durchgeführt werden. Es handelt sich dabei um eine Hydrolyse der Nebenprodukte zu Mesityloxid und/oder Aceton. Der dazu eingesetzte Temperaturbereich ist insbesondere < 205 °C, bevorzugt < 204 °C, liegt bevorzugter im Bereich von 30 °C bis 200 °C, liegt noch bevorzugter im Bereich von 70 °C bis 185 °C.

Bevorzugt wird gemäß Schritt (d) des erfindungsgemäßen Verfahrens das Wasser zu **RP1** zugegeben, während in Schritt (b) Aceton und/oder Mesityloxid aus **RP1** mindestens teilweise abgetrennt werden.

Noch bevorzugter ist diese Ausführungsform dann, wenn die mindestens teilweise destillative Abtrennung von Aceton und/oder Mesityloxid aus **RP1** in Schritt (b) in einer Destillationskolonne erfolgt.

Noch viel mehr bevorzugter erfolgt die Umsetzung des zugegebenen Wassers in Schritt (d) mit mindestens einem der Nebenprodukte in **RP1** ausgewählt aus der Gruppe bestehend aus Phoron, Diacetonalkohol, Diacetonamin, Acetonin, Isophoron, bevorzugt ausgewählt aus der Gruppe bestehend aus Diacetonalkohol, Diacetonamin, Acetonin, Isophoron, zu Aceton und/oder Mesityloxid dann in der Gasphase.

In diesem Fall wird insbesondere das Wasser in die Destillationskolonne während der destillativen Abtrennung, etwa über einen Zulauf von Wasser in die Destillationskolonne oder durch Zugabe von Wasserdampf zur Destillationskolonne, zugeführt. Die Umsetzung mindestens eines der Nebenprodukte Diacetonalkohol, Diacetonamin, Acetonin, Phoron, Isophoron, bevorzugt der Nebenprodukte ausgewählt aus der Gruppe bestehend aus Diacetonalkohol, Diacetonamin, Acetonin, Isophoron, mit Wasser zu Aceton und/oder Mesityloxid findet dann in der Destillationskolonne statt.

Diese Ausführungsform hat sich als besonders vorteilhaft erwiesen, da diese Ausführungsform besonders gut gewährleistet, dass gezielt die Nebenkomponenten zersetzt werden und die unerwünschte Rückreaktion des TAA unterdrückt wird. Dies wird besonders gut dann gewährleistet und es ist deshalb bevorzugt, wenn der bei der Destillation in Schritt (b) eingesetzte Temperaturbereich unterhalb der Siedetemperatur von TAA, wobei der Druck insbesondere Normaldruck ist, liegt, bevorzugt < 205 °C bei Normaldruck beträgt, bevorzugter < 204 °C bei Normaldruck beträgt, noch bevorzugter im Bereich von 30 °C bis 200 °C bei Normaldruck liegt, noch mehr bevorzugter im Bereich von 70 °C bis 185 °C bei Normaldruck liegt.

### 4. Schritt (c)

Im Schritt (c) des erfindungsgemäßen Verfahrens wird dann Ammoniak und gegebenenfalls Aceton zu dem in Schritt (b) aus **RP1** abgetrennten Aceton und/oder Mesityloxid gegeben und Aceton und/oder Mesityloxid mindestens teilweise mit dem zugegebenen Ammoniak zu TAA in Gegenwart eines Katalysators **K2** umgesetzt.

Dabei wird wieder ein Rohprodukt **RP2** umfassend Triacetonamin erhalten. Dieses umfasst insbesondere neben dem gewünschten Produkt TAA auch nicht reagiertes Aceton sowie als Nebenprodukt Mesityloxid sowie die höhermolekulare Kondensationsprodukte des Acetons mit sich oder Ammoniak. Dies sind vor allem Nebenprodukte ausgewählt aus der Gruppe bestehend aus Diacetonalkohol, Diacetonamin, Acetonin, Phoron, Isophoron, bevorzugt ausgewählt aus der Gruppe bestehend aus Diacetonalkohol, Diacetonamin, Acetonin, Isophoron.

In Schritt (c) des erfindungsgemäßen Verfahrens wird dabei insbesondere so viel Ammoniak zugegeben, dass für jedes Mol in Schritt (c) eingesetzten Acetons 0.05 bis 0.33 Mol, bevorzugt 0.10 bis 0.166 Mol, bevorzugter 0.143 Mol Ammoniak zugegeben werden.

Zusätzlich zu diesem für Aceton zugegeben Ammoniak wird insbesondere für jedes Mol in Schritt (c) eingesetzten Mesityloxids 0.1 bis 0.66 Mol, bevorzugt 0.25 bis 0.33 Mol, bevorzugter 0.286 Mol Ammoniak zugegeben.

Die Umsetzung in Schritt (c) kann in Gegenwart weiterer Lösungsmittel oder nur in Aceton, also ohne Zugabe weiterer Lösungsmittel, stattfinden. In den Fällen, in denen ein Lösungsmittel in Schritt (c) eingesetzt wird, können sämtliche Lösungsmittel eingesetzt werden, die die Reaktion nicht verhindern. Insbesondere sind die möglichen Lösungsmittel aliphatische Lösungsmittel, bevorzugt Pentan, Hexan, Heptan, Octan, Decan, Cyclohexan, Tetramethylsilan; aromatische Lösungsmittel, bevorzugt Benzol, Toluol, Xylol; Ether-Verbindungen, bevorzugt Diethylether, Dipropylether, Dibutylether, Methyl-*tert*-Butylether; halogenierte Lösungsmittel, bevorzugt Dichlormethan, Chloroform, Tetrachlormethan; Alkohole, bevorzugt Methanol, Ethanol, Propanol, Isopropanol, Butanol, *tert*-Butanol; Ester, bevorzugt Methylacetat, Ethylacetat, Propylacetat, Butylacetat. Besonders bevorzugt findet die Umsetzung in Schritt (c) in Aceton, ohne Zugabe weiterer Lösungsmittel, statt.

Im kontinuierlichen Betrieb werden bevorzugt alle Chemikalien gleichzeitig bei der Reaktionstemperatur zudosiert. Bei der kontinuierlichen Reaktion kann jeder dem Fachmann bekannte Reaktor eingesetzt werden, z.B. ein kontinuierliches Strömungsrohr, ein kontinuierlicher Rührkessel, eine Rührkesselkaskade, sowie mögliche Kombinationen aus diesen einzelnen Elementen. Dabei wird bevorzugt eine Kombination von einem oder mehreren Reaktoren mit internem oder externem Kreislauf, gefolgt von einem Nachreaktor mit Strömungsrohrcharakteristik, eingesetzt.

Die Reaktionszeit in Schritt (c) im Batch-Betrieb liegt im Bereich von 1 bis 15 Stunden, bevorzugt im Bereich von 3 bis 9 Stunden, besonders bevorzugt im Bereich von 5 bis 7 Stunden. Die Reaktionszeiten für die kontinuierliche Verfahrensführung ergeben sich durch die Gesamt-Verweilzeit der Reaktanden im Reaktor und liegen in dem für den Batch-Betrieb genannten Bereich.

Die *"volume space velocity"* für Ammoniak im kontinuierlichen Betrieb liegt in Schritt (c) insbesondere bei 0.01 bis 124.20 h⁻¹, bevorzugt 0.03 bis 5.25 h⁻¹, am bevorzugtesten bei 0.06 h⁻¹ (dies entspricht dem Volumen an Ammoniak, was pro Stunde und pro Volumen des Reaktors durch diesen Reaktor fließt).

Die *"volume space velocity"* für Aceton im kontinuierlichen Betrieb liegt in Schritt (c) insbesondere bei 0.15 bis 1.33 h⁻¹, bevorzugt 0.66 bis 1.17 h⁻¹ (dies entspricht dem Volumen an Aceton, was pro Stunde und pro Volumen des Reaktors durch diesen Reaktor fließt).

Die Umsetzung in Schritt (c) wird vorzugsweise bei erhöhter Temperatur durchgeführt, insbesondere bei Temperaturen im Bereich von 20 °C bis 180°C, bevorzugt im Bereich von 40 °C bis 100 °C, besonders bevorzugt im Bereich von 55 °C bis 90°C, noch bevorzugter im Bereich von 60 °C bis 90 °C, noch bevorzugter bei 75 °C.

Die Umsetzung in Schritt (c) wird insbesondere entweder bei Eigendruck der Komponenten oder bei erhöhtem Druck durchgeführt werden. So wird die Umsetzung in Schritt (c) bevorzugt bei einem Druck im Bereich von 1 bis 16 bar, bevorzugter bei einem Druck im Bereich von 1 bis 15 bar, noch bevorzugter bei einem Druck im Bereich von 7 bis 14 bar, noch mehr bevorzugter bei 12 bar durchgeführt.

Ammoniak wird im Schritt (c) des erfindungsgemäßen Verfahren bevorzugt als Reinstoff, d.h. als Gas zudosiert und liegt insbesondere während der Reaktion gelöst in Aceton bzw. gelöst im Reaktionsgemisch vor.

Im Schritt (c) des erfindungsgemäßen Verfahrens wird das in Schritt (b) des erfindungsgemäßen Verfahrens aus **RP1** abgetrennte Aceton und/oder Mesityloxid als Edukt eingesetzt. Um die Reaktion vorteilhaft weiter auf Seite des TAA zu schieben, kann in Schritt (c) des erfindungsgemäßen Verfahrens zusätzliches Aceton zugegeben werden.

Der Schritt (c) des erfindungsgemäßen Verfahrens wird in Gegenwart eines Katalysators **K2** durchgeführt. Als Katalysator **K2** sind alle im Stand der Technik für diesen Reaktionstyp genannten Katalysatoren einsetzbar. Der Katalysator **K2** kann dabei homogen oder heterogen sein, ist bevorzugt jedoch heterogen.

Als homogener Katalysator **K2** sind alle im Stand der Technik für diesen Reaktionstyp beschriebenen homogenen Katalysatoren geeignet, z.B. Brönstedt-Säuren, Salze dieser Säuren oder Lewissäuren, wie sie in der EP 2 706 056 A1 beschrieben sind.

Vorzugsweise ist in den Fällen, in denen **K2** ein homogene Katalysator ist, dieser ausgewählt aus der Gruppe der Ammoniumsalze, insbesondere aus der Gruppe umfassend Salze aus Ammoniak und starken Brönsted-Säuren [z.B. Salzsäure, Schwefelsäure, Salpetersäure, organische Säuren (R**COOH) oder Sulfonsäuren (R**SO₃H), wobei R** ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten].

Bevorzugt ist in den Fällen, in denen **K2** ein homogener Katalysator ist, dieser dann Ammoniumnitrat. Ammoniumnitrat hat den Vorteil, dass es günstig, nicht toxisch, halogenidfrei und damit weniger korrosiv ist.

Bevorzugt wird als Katalysator **K2** jedoch ein heterogener Katalysator, insbesondere ein fester, saurer Katalysator eingesetzt, wie er beispielsweise in der DE 28 07 172 A1, der CN 103224465 A oder der DE 10 2010 062 804 A1 beschrieben ist. Dies sind solche Katalysatoren, die in dem Reaktionsmedium praktisch unlöslich sind. Bevorzugt wird dazu ein Katalysator genutzt, der anorganisch oder organisch ist und aktive saure Gruppen, bevorzugt Sulfonsäureestergruppen oder Phosphorsäureestergruppen, aufweist.

**K2** ist demnach insbesondere ausgewählt aus der Gruppe bestehend aus Aluminiumhydrosilikate vom Betonit- und/oder Montmorillonit-Typ, anorganische Ionenaustauscher auf Aluminiumsilikat-Basis vom Zeolithtyp, Mordenittyp, Erionittyp oder auch mit Phosphorsäure bei 700 bis 1100 °C, wie in CA 772 201 beschrieben, behandelte Diatomerde.

Besonders für **K2** bevorzugte heterogene Katalysatoren sind lonenaustauscherharze, insbesondere Kationenaustauscherharze. Diese sind bevorzugt sauer.

Als lonenaustauscherharze für **K2** kommen insbesondere solche auf anorganischer (beispielsweise Siliciumdioxid) oder organischer (beispielsweise Polystyrol oder Polystyrolcopolymere, wie Styrol-Divinylbenzol-Copolymere), bevorzugt organischer, Basis, die protische Säuregruppen, insbesondere Alkylsulfonsäureestergruppen, Sulfonsäureestergruppen (-SO₃⁻), Phosphorsäureestergruppen, insbesondere Sulfonsäureestergruppen aufweisen, in Frage.

Ein besonders bevorzugter heterogener Katalysator für **K2** ist ausgewählt aus der folgenden Gruppe:
- Ein Katalysator, der ein Polymerharz, insbesondere Polystyrol oder Styrol-Divinylbenzol-Copolymer, bevorzugt Polystyrol, und protische Säuren, insbesondere -SO₃⁻-Gruppen, als funktionelle Gruppen aufweist (käuflich erwerblich als "Amberlyst® 15", "Amberlite® 200", "Lewatit® SP 120" bzw. "Lewatit® K2621"); daneben ist auch Polystyrolsulfonat mit der CAS-Nummer: 28210-41-5 einsetzbar;
- Ein Katalysator, der protische Säuren, insbesondere Sulfonsäure in polymerer Form aufweist und perfluoriert sein kann (beschrieben in DE 10 2010 062 804 A1, US 4,831,146). Dieser kann zum Beispiel ein sulfoniertes Tetrafluorethylen (CAS-Nummer: 31175-20-9) oder eine feste geträgerte perfluorierte Sulfonsäure in polymerer Form mit Siliciumdioxid als Trägermaterial sein. Derartige Katalysatoren sind unter anderem unter den Handelsnamen Nafion®, Aciplex® F, Femion®, Neosepta®, Fumion® F erhältlich. Ein bevorzugter Katalysator ist Nafion® SAC-13. Bei Nafion® SAC-13 handelt es sich um poröse Siliciumdioxidpartikel, auf dem Nafion® in einer Beladung von etwa 13 Gew.-% adsorbiert worden ist;
- Poly(2-acrylamido-2-methyl-1-propanesulfonsäure), vertrieben als PolyAMPS® von Lubrizol.

Am bevorzugtesten wird als **K2** ein heterogener Katalysator eingesetzt, der ein Polymerharz, insbesondere Polystyrol oder Styrol-Divinylbenzol-Copolymer, insbesondere Polystyrol, und protische Säuren, insbesondere -SO₃⁻-Gruppen, als funktionelle Gruppen aufweist (käuflich erwerblich als "Amberlyst® 15", "Amberlite® 200", "Lewatit® SP 120" bzw. "Lewatit® K2621").

Die in Schritt (c) eingesetzte Menge an Katalysator **K2** ist nicht besonders beschränkt und kann vom Fachmann bestimmt werden. Typischerweise kann, wenn der Katalysator ein homogener aus der Gruppe der Brönstedt-Säuren, Salze dieser Säuren oder Lewissäuren, bevorzugt ein Ammoniumsalz, noch bevorzugter Ammoniumnitrat ist, dieser im Molverhältnis von Ammoniak zu Katalysator, vorzugsweise Ammoniumnitrat, im Bereich von 1 : 0.8 bis 1 : 0.02 eingesetzt werden. Ganz besonders bevorzugt liegt das Molverhältnis von Aceton : Ammoniak : Ammoniumnitrat im Bereich von 7 bis 8 : 0.9 bis 1.1 : 0.085 bis 0.098.

In der bevorzugten Ausführungsform, in welcher ein fester, saurer Ionenaustauscher für **K2** eingesetzt wird, kann dieses als Festbett eingesetzt werden, beispielsweise in einer Menge von 10 bis 20 Vol.-% bezogen auf die Gesamtmenge des in Schritt (c) eingesetzten Acetons und Mesityloxids.

In Schritt (c) des erfindungsgemäßen Verfahrens wird dann ein Rohprodukt **RP2** erhalten, welches das gewünschte Produkt Triacetonamin und gegebenenfalls auch das ursprünglich eingesetzte Edukt Aceton und/oder Mesityloxid, sowie insbesondere mindestens ein Nebenprodukt ausgewählt aus der Gruppe bestehend Diacetonalkohol, Diacetonamin, Acetonin, Phoron, Isophoron, bevorzugt ausgewählt aus der Gruppe bestehend aus Diacetonalkohol, Diacetonamin, Acetonin, Isophoron, umfasst.

Es versteht sich von selbst, dass **RP2** dann wiederum einem dem Reaktionsschritt (b) des erfindungsgemäßen Verfahrens entsprechenden Reaktionsschritt unterworfen werden kann.

In einer bevorzugten Ausführungsform des erfindungsgemäßen Verfahrens sind der Katalysator **K1** in Schritt (a) und der Katalysator **K2** in Schritt (c) des erfindungsgemäßen Verfahrens unabhängig voneinander ein heterogener, noch bevorzugter fester, saurer Katalysator, bevorzugter wie sie oben definiert wurden. Am allerbevorzugtesten sind **K1** und **K2** gleich.

### 5. Abtrennung von TAA

Die Abtrennung von TAA aus **RP1** bzw. **RP2** kann im Übrigen durch Kristallisation, Destillation, bevorzugt mit Destillation, welche noch bevorzugter in einer nachfolgenden Destillationskolonne stattfindet, erfolgen.

Die nachfolgenden Beispiele sollen die Erfindung illustrieren, ohne diese zu beschränken.

### Beispiele

### 1. Umsetzung

Zwei in Reihe geschaltete zylindrische Reaktoren wurden mit Lewatit K2621 (Polystyrol-Katalysator mit -SO₃⁻ als funktionelle Gruppe; von Lanxess) so gefüllt, dass dieses Katalysatormaterial durch Siebe oben und unten begrenzt, angeordnet war. Das Schüttvolumen des Katalysators betrug je 600 mL im wasserfeuchten Zustand. Der Reaktor wurde kontinuierlich mit 630 g/h Aceton und 25 g/h Ammoniak beschickt. Dabei wurden eine Temperatur von 75 °C und ein Druck von 14 bar eingestellt. Der Zulauf betrug insgesamt 961.83 g (16.58 mol) Aceton. Die LHSV (liquid hourly space velocity, also das Volumen an Reaktanden die pro Stunde bezogen auf das Reaktorvolumen zugegeben wurden), betrug im Falle des Ammoniaks 0.03 bis 0.06 h⁻¹, und im Falle des Acetons 0.66 bis 1.33 h⁻¹.

### 2. E1: Aufarbeitung gemäß erfindungsgemäßen Verfahren, d.h. mit Hydrolyse

1 kg des Reaktoraustrages aus Versuch gemäß Punkt 1 wurde mit 3 bis 5 Gew.-% Wasser, bezogen auf die unter Punkt 1 eingesetzte Menge an Aceton, versetzt und anschließend destillativ an einer Rektifikationskolonne aufgearbeitet. Dabei wurde so lange destilliert, dass eine Abtrennung sämtlicher Leichtsieder (Aceton, Mesityloxid, Diacetonalkohol, Diacetonamin) gelang, TMDHPyridin und TAA und weitere entstandene Hochsieder jedoch als Abfall- bzw. Wertprodukt über den Sumpf abgetrennt wurden. Zwischensieder, wie Acetonin und Phoron, wurden zu einem möglichst hohen Anteil in leichtsiedende Komponenten hydrolisiert und waren dadurch abtrennbar.

Danach wurde der Anteil an den verschiedenen Komponenten im Sumpf und im Destillat per GC [Säule: HP-50 (30m), Temperaturprogramm: 5 °C/min 60 - 130°C, 15 °C/min - 270°C, 10 min] bestimmt. Der relative Anteil der jeweiligen Komponenten im Reaktoraustrag, Destillat und Sumpf wurde bestimmt und ist in der folgenden Tabelle 1 aufgeführt. In der folgenden Tabelle 1 sind (mit Ausnahme des Wassers) die Komponenten im Reaktionsaustrag von links nach rechts gemäß ihrer Siedepunkte aufgeführt ("n.i." = nicht identifiziert).

Die ersten drei Zeilen geben die Flächenprozente (inklusive Wasser) der jeweiligen Komponente im GC-Diagramm wieder.

Die letzte Zeile ergibt sich wie folgt:
Basierend auf der Gesamtmasse des Sumpfes (200 g) wurden die Massen der jeweiligen Komponente im Sumpf aus dem prozentualen Anteil der jeweiligen Verbindung im GC ermittelt. Daraus lässt sich die Stoffmenge der jeweiligen Komponente im Sumpf und somit auch die Stoffmenge an Acetonäquivalenten berechnen, denen die jeweilige Komponente entspricht.

Dabei entspricht Aceton einem Acetonäquivalent.
Dabei entsprechen die folgenden Verbindungen zwei Acetonäquivalenten: Mesityloxid (und Isomer), Diacetonalkohol, Diacetonamin.

Dabei entsprechen die folgenden Verbindungen drei Acetonäquivalenten: Acetonin, Phoron, Isophoron.

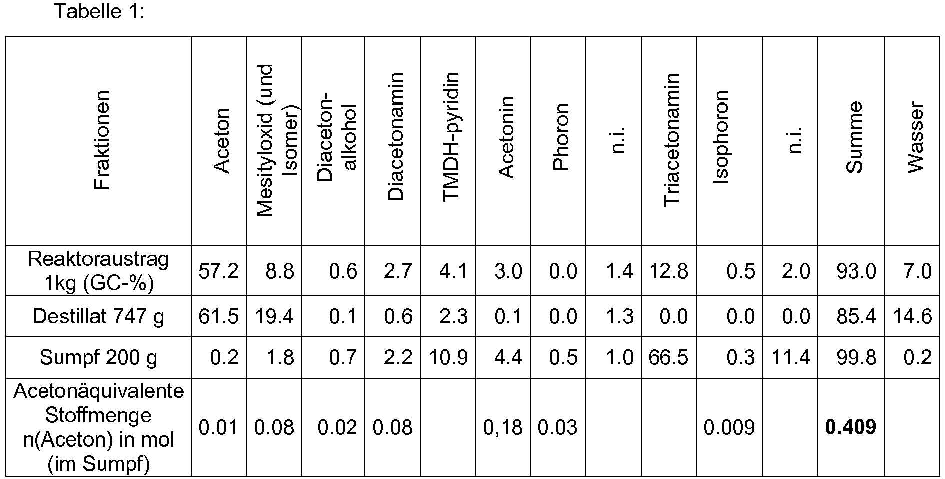

Der Anteil an Acetonäquivalenten, den man bei der erfinderischen Vorgehensweise bezogen auf den eingesetzten Anteil an Aceton (16.58 mol) verliert, wurde so ermittelt und betrug 2.5 %.

### 3. V1: Vergleichsversuch ohne Hydrolyse

Versuch **E1** wurde wiederholt, außer dass keine Zugabe von Wasser stattfand.
Die entsprechenden Gehalte der jeweiligen Komponenten im Reaktoraustrag, Destillat und Sumpf sowie die Stoffmenge an Acetonäquivalenten im Sumpf wurden wie für **E1** beschrieben ermittelt und sind in der folgenden Tabelle 2 aufgeführt ("n.i." = nicht identifiziert).

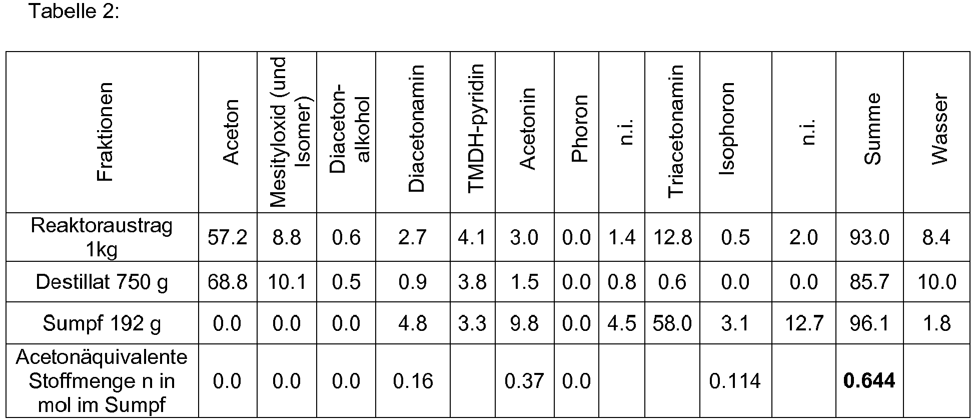

Es wurde demnach ermittelt, dass sich 3.9 % der eingesetzten Acetonäquivalente im Sumpf befanden und somit für weitere Reaktionsschritte mit Ammoniak verloren waren.

### 4. Ergebnisse

4.1) Bei **E1** (Destillat: 747 g; Sumpf 200 g) und bei **V1** (Destillat: 750 g; Sumpf: 192 g) konnte fast der gleiche Anteil an Destillat und Sumpf rückgewonnen werden;
4.2) Der Gehalt an TAA im Sumpf war höher bei **E1** als bei **V1**, das heißt die absolute Ausbeute an TAA war im Fall des erfindungsgemäßen Vorgehens besser.
4.3) Der Anteil an nicht im Sumpf verlorenen und somit reaktiven Acetonäquivalenten ist beim erfindungsgemäßen Vorgehen gemäß **E1** höher. Die Tatsache, dass in **E1** (Tabelle 1) noch ein Anteil an Aceton, Mesityloxid und Diacetonalkohol im Sumpf gefunden wurde, bei gleichzeitigem verringertem Anteil an Acetonin und Isophoron weist außerdem darauf hin, dass bei der erfindungsgemäßen Vorgehensweise offensichtlich diese höhermolekularen Nebenprodukte in Aceton bzw. Mesityloxid gespalten wurden, während dies bei dem herkömmlichen Vorgehen (**V2**; Tabelle 2) nicht der Fall ist. Entscheidend ist, dass der Gesamtgehalt an Acetonäquivalenten im Sumpf bei **V1** höher ist als der Gesamtgehalt an Acetonäquivalenten im Sumpf bei **E1**.

## Patentansprüche

1. Verfahren zur Herstellung von Triacetonamin umfassend die folgenden Schritte:
(a) Umsetzung von Aceton und Ammoniak in Gegenwart eines Katalysators **K1** zu einem Rohprodukt **RP1** umfassend Triacetonamin, Aceton, Mesityloxid und mindestens ein Nebenprodukt ausgewählt aus der Gruppe bestehend aus Diacetonalkohol, Diacetonamin, Acetonin, Phoron, Isophoron,
(b) mindestens teilweise destillative Abtrennung von Aceton und/oder Mesityloxid aus **RP1**,
(c) Zugabe von Ammoniak und gegebenenfalls weiteren Acetons zu dem in Schritt (b) aus **RP1** abgetrennten Aceton und/oder Mesityloxid und mindestens teilweise Umsetzung des zugegebenen Ammoniaks mit Aceton und/oder Mesityloxid zu TAA in Gegenwart eines Katalysators **K2**, wodurch ein Rohprodukt **RP2** umfassend Triacetonamin erhalten wird,
**dadurch gekennzeichnet, dass**
(d) vor und/oder während der in Schritt (b) stattfindenden destillativen Abtrennung von Aceton und/oder Mesityloxid aus **RP1** Wasser zu **RP1** zugegeben wird und mindestens eines der Nebenprodukte in **RP1** ausgewählt aus der Gruppe bestehend aus Diacetonalkohol, Diacetonamin, Acetonin, Phoron, Isophoron mindestens teilweise mit Wasser umgesetzt wird, so dass mindestens eines der Nebenprodukte in **RP1** mindestens teilweise in Mesityloxid und/oder Aceton gespalten wird.

2. Verfahren nach Anspruch 1, wobei im Schritt (d) während der in Schritt (b) stattfindenden destillativen Abtrennung von Aceton und/oder Mesityloxid aus **RP1** Wasser zu **RP1** zugegeben wird und mindestens eines der Nebenprodukte in **RP1** ausgewählt aus der Gruppe bestehend aus Diacetonalkohol, Diacetonamin, Acetonin, Phoron, Isophoron mindestens teilweise mit Wasser umgesetzt wird, so dass mindestens eines der Nebenprodukte in **RP1** mindestens teilweise in Mesityloxid und/oder Aceton gespalten wird.

3. Verfahren nach einem der Ansprüche 1 oder 2, wobei die in Schritt (b) stattfindende mindestens teilweise destillative Abtrennung von Aceton und/oder Mesityloxid aus **RP1** in einer Destillationskolonne erfolgt.

4. Verfahren nach Anspruch 3, wobei der bei der Destillation in Schritt (b) eingesetzte Temperaturbereich unterhalb der Siedetemperatur von Triacetonamin liegt.

5. Verfahren nach einem der Ansprüche 1 bis 4, wobei der Schritt (a) bei einer Temperatur von 20 °C bis 180 °C durchgeführt wird.

6. Verfahren nach einem der Ansprüche 1 bis 5, wobei das Molverhältnis von in Schritt (a) eingesetztem Aceton zu in Schritt (a) eingesetztem Ammoniak 3 : 1 bis 20 : 1 beträgt.

7. Verfahren nach einem der Ansprüche 1 bis 6, wobei **K1** ein heterogener Katalysator ist.

8. Verfahren nach einem der Ansprüche 1 bis 7, wobei die Menge des in Schritt (d) zugegebenen Wassers ≥ 0.1 Gew.-%, bezogen auf die Summe der Gewichte des von **RP1** umfassten Phorons, Diacetonalkohols, Diacetonamins, Acetonins und Isophorons beträgt.

9. Verfahren nach einem der Ansprüche 1 bis 8, wobei die Umsetzung in Schritt (c) bei einer Temperatur von 20 °C bis 180 °C durchgeführt wird.

10. Verfahren nach einem der Ansprüche 1 bis 9, wobei **K2** ein heterogener Katalysator ist.

11. Verfahren nach einem der Ansprüche 1 bis 10, wobei in Schritt (c) insgesamt so viel Ammoniak zugegeben wird, dass für jedes Mol in Schritt (c) eingesetzten Acetons 0.05 bis 0.33 Mol Ammoniak zugegeben werden, und zusätzlich zu diesem für Aceton zugegeben Ammoniak für jedes Mol in Schritt (c) eingesetzten Mesityloxids 0.1 bis 0.66 Mol Ammoniak zugegeben werden.

## Claims

1. Method for preparing triacetone amine, comprising the following steps:
(a) reaction of acetone and ammonia in the presence of a catalyst **K1** to give a crude product **RP1** comprising triacetone amine, acetone, mesityl oxide and at least one by-product selected from the group consisting of diacetone alcohol, diacetone amine, acetonin, phorone, isophorone,
(b) at least partial distillative removal of acetone and/or mesityl oxide from **RP1**,
(c) addition of ammonia and optionally further acetone to the acetone and/or mesityl oxide removed from **RP1** in step (b), and at least partial reaction of the added ammonia with acetone and/or mesityl oxide in the presence of a catalyst **K2** to give TAA, as a result of which a crude product **RP2** comprising triacetone amine is obtained,
**characterized in that**
(d) before and/or during the distillative removal of acetone and/or mesityl oxide from **RP1** occurring in step (b), water is added to **RP1** and at least one of the by-products in **RP1**, selected from the group consisting of diacetone alcohol, diacetone amine, acetonin, phorone, isophorone, is at least partially reacted with water, such that at least one of the by-products in **RP1** is at least partially cleaved into mesityl oxide and/or acetone.

2. Method according to Claim 1, wherein, in step (d), during the distillative removal of acetone and/or mesityl oxide from **RP1** occurring in step (b), water is added to **RP1** and at least one of the by-products in **RP1**, selected from the group consisting of diacetone alcohol, diacetone amine, acetonin, phorone, isophorone, is at least partially reacted with water, such that at least one of the by-products in **RP1** is at least partially cleaved into mesityl oxide and/or acetone.

3. Method according to either of Claims 1 and 2, wherein the at least partial distillative removal of acetone and/or mesityl oxide from **RP1** occurring in step (b) takes place in a distillation column.

4. Method according to Claim 3, wherein the temperature range used in the distillation in step (b) is below the boiling point of triacetone amine.

5. Method according to one of Claims 1 to 4, wherein step a) is carried out at a temperature of 20°C to 180°C.

6. Method according to one of Claims 1 to 5, wherein the molar ratio of acetone used in step (a) to ammonia used in step (a) is 3 : 1 to 20 : 1.

7. Method according to one of Claims 1 to 6, wherein **K1** is a heterogeneous catalyst.

8. Method according to one of Claims 1 to 7, wherein the amount of water added in step (d) is ≥ 0.1 wt%, based on the sum of the weights of phorone, diacetone alcohol, diacetone amine, acetonin and isophorone contained in **RP1.**

9. Method according to one of Claims 1 to 8, wherein the reaction in step (c) is carried out at a temperature of 20°C to 180°C.

10. Method according to one of Claims 1 to 9, wherein **K2** is a heterogeneous catalyst.

11. Method according to one of Claims 1 to 10, wherein, in step (c), ammonia is added in an overall amount such that, for each mole of acetone used in step (c), 0.05 to 0.33 mol of ammonia is added, and in addition to this ammonia added for acetone, for each mole of mesityl oxide used in step (c), 0.1 to 0.66 mol of ammonia is added.

## Revendications

1. Procédé pour la préparation de la triacétonamine comprenant les étapes suivantes :
(a) transformation d'acétone et d'ammoniac en présence d'un catalyseur **K1** pour donner un produit brut **RP1** comprenant de la triacétonamine, de l'acétone, de l'oxyde de mésityle et au moins un sous-produit choisi dans le groupe constitué par le diacétone alcool, la diacétonamine, l'acétonine, la phorone, l'isophorone,
(b) séparation par distillation au moins partielle d'acétone et/ou d'oxyde de mésityle de **RP1**,
(c) ajout d'ammoniac et éventuellement d'acétone supplémentaire à l'acétone et/ou à l'oxyde de mésityle séparé(e) de **RP1** dans l'étape (b) et transformation au moins partielle de l'ammoniac ajouté avec l'acétone et/ou l'oxyde de mésityle pour donner la TAA en présence d'un catalyseur **K2**, un produit brut **RP2** comprenant de la triacétonamine étant ainsi obtenu,
**caractérisé en ce que**
(d) avant et/ou pendant la séparation par distillation d'acétone et/ou d'oxyde de mésityle de **RP1** ayant lieu dans l'étape (b), de l'eau est ajoutée à **RP1** et au moins l'un des sous-produits dans **RP1** choisi dans le groupe constitué par le diacétone alcool, la diacétonamine, l'acétonine, la phorone, l'isophorone est transformé au moins partiellement avec de l'eau, de sorte qu'au moins l'un des sous-produits dans **RP1** est au moins partiellement fragmenté en oxyde de mésityle et/ou en acétone.

2. Procédé selon la revendication 1, dans lequel dans l'étape (d), pendant la séparation par distillation d'acétone et/ou d'oxyde de mésityle de **RP1** ayant lieu dans l'étape (b), de l'eau est ajoutée à **RP1** et au moins l'un des sous-produits dans **RP1** choisi dans le groupe constitué par le diacétone alcool, la diacétonamine, l'acétonine, la phorone, l'isophorone est transformé au moins partiellement avec de l'eau, de sorte qu'au moins l'un des sous-produits dans **RP1** est au moins partiellement fragmenté en oxyde de mésityle et/ou en acétone.

3. Procédé selon l'une quelconque des revendications 1 et 2, dans lequel la séparation par distillation au moins partielle d'acétone et/ou d'oxyde de mésityle de **RP1** ayant lieu dans l'étape (b) est réalisée dans une colonne de distillation.

4. Procédé selon la revendication 3, le domaine de température utilisé lors de la distillation dans l'étape (b) se situant en dessous de la température d'ébullition de la triacétonamine.

5. Procédé selon l'une quelconque des revendications 1 à 4, l'étape (a) étant mise en œuvre à une température de 20 °C à 180 °C.

6. Procédé selon l'une quelconque des revendications 1 à 5, le rapport molaire de l'acétone utilisée dans l'étape (a) sur l'ammoniac utilisé dans l'étape (a) étant de 3:1 à 20:1.

7. Procédé selon l'une quelconque des revendications 1 à 6, **K1** étant un catalyseur hétérogène.

8. Procédé selon l'une quelconque des revendications 1 à 7, la quantité d'eau ajoutée dans l'étape (d) étant ≥ 0,1% en poids, par rapport à la somme des poids de la phorone, du diacétone alcool, de la diacétonamine, de l'acétonine et de l'isophorone compris dans **RP1**.

9. Procédé selon l'une quelconque des revendications 1 à 8, la transformation dans l'étape (c) étant mise en œuvre à une température de 20 °C à 180 °C.

10. Procédé selon l'une quelconque des revendications 1 à 9, **K2** étant un catalyseur hétérogène.

11. Procédé selon l'une quelconque des revendications 1 à 10, dans lequel dans l'étape (c) autant d'ammoniac est ajouté au total afin que pour chaque mole d'acétone utilisée dans l'étape (c), 0,05 à 0,33 mole d'ammoniac soit ajoutée, et qu'en plus de cet ammoniac ajouté pour l'acétone, pour chaque mole d'oxyde de mésityle utilisé dans l'étape (c), 0,1 à 0,66 mole d'ammoniac soit ajoutée.
